# EUROPÄISCHE PATENTSCHRIFT

(11) **EP 1 581 506 B1**
(45) Veröffentlichungstag und Bekanntmachung des Hinweises auf die Patenterteilung: **29.11.2006**
(21) Anmeldenummer: 04700454.4
(22) Anmeldetag: 07.01.2004
(51) Int. Cl.: C07D 239/84

(54) **VERWENDUNG VON 2-AMINO-2H-CHINAZOLIN-DERIVATEN ZUR HERSTELLUNG VON THERAPEUTISCHEN MITTELN**
USE OF 2-AMINO-2H-QUINAZOLINE DERIVATIVES FOR PRODUCING THERAPEUTIC AGENTS
UTILISATION DE DERIVES DE 2-AMINO-2H-QUINAZOLINE POUR LA PREPARATION DE PRODUITS THERAPEUTIQUES

(30) Priorität: 09.01.2003 DE 10301105
(43) Veröffentlichungstag der Anmeldung: 05.10.2005
(73) Patentinhaber: Chemisch-Pharmazeutisches Labor Rolf Sachse GmbH, 13627 Berlin (DE)
(72) Erfinder: SACHSE, Rolf, 13465 Berlin (DE)
(74) Vertreter: Schubert, Klemens
(86) Internationale Anmeldenummer: PCT/EP2004/000054
(87) Internationale Veröffentlichungsnummer: WO 2004/063172

(56) Entgegenhaltungen:
- WO-A-01/40196
- US-A- 3 932 407
- US-A- 4 146 718
- US-B1- 6 388 073

## Beschreibung

Die vorliegende Erfindung betrifft die Verwendung von 2-Amino-2H-chinazolin-Derivaten sowie von deren pharmazeutisch verträglichen Salzen zur Herstellung von therapeutischen Mitteln zur Behandlung von myeloproliferativen Erkrankungen und Bronchodilation.

Myeloproliferative Erkrankungen gehören zu den Erkrankungen, welche das Knochenmark betreffen. Das gemeinsame Merkmal der myeloproliferativen Erkrankungen ist eine selbstständige Vermehrung aller Blutzellen des Knochenmarks (rote und weiße Blutkörperchen sowie Blutplättchen). Zu ihnen gehören unter anderen die Polycythämia vera und essentielle Thrombozythämie. Erstere kann durch Thrombozytosen mit wechselndem Ausmaß begleitet sein.

Thrombozythämie ist eine bösartige Erkrankung des Knochenmarks, die durch eine erhöhte Anzahl der Blutplättchen (Thrombozyten) im Blut gekennzeichnet ist. Die Ursache für die Thrombozythämie ist ein Defekt der Stammzelle der Blutbildung, die der Ursprung aller Blutzellen ist und im Knochenmark gebildet wird. Die großen Vorläuferzellen der Thrombozyten sind die Megakaryozyten, welche bei der Thrombozythämie in starkem Maße gebildet werden. Beim Platzen eines einzigen Megakaryozyten werden mehrere tausend kernlose Blutplättchen freigesetzt. Bei der Behandlung von myeloproliferativen Erkrankungen, wie der essentiellen Thrombozythämie, wird daher neben anderen die Verbindung 6,7-Dichlor-1,5-dihydroimidazo-[2,1-b]-chinazolin-2-[3H]-on eingesetzt, besser bekannt unter dem Namen Anagrelid (vorzugsweise als Hydrochlorid verwendet) (z.B.: Anagrelide: a new drug for treating thrombocytosis (Silverstein, M.N. et al. *N Engl J Med* (1988) 318(20): 1292-4). Anagrelid ist eine Substanz, die sich durch eine selektive thrombozytensenkende Wirkung auszeichnet, beispielsweise beschrieben von Lindley et al. (Anagrelide: a novel agent for the treatment of myeloproliferative disorders. Pescatore SL, Lindley C *Expert Opin Pharmacother* (2000) 1(3): 537-46).

Die Gabe von beispielsweise 3 mg/Tag verursacht nach einer Woche einen Thrombozytenabfall um 50 Prozent, wobei Anagrelid über einen noch unbekannten Hemmeffekt in die Megakaryozytopoese eingreifen soll.

Andere Wirkungen von Anagrelid sind in US-A-3,932,407 und US-A-4,146,718 beschrieben, so bei der Verwendung als Blutplättchen anti-aggregatives oder blutdrucksenkendes Mittel und bei der Behandlung zur Bronchodilation. In US-A-4,146,718 wird auch eine Blutplättchen anti-aggregative Wirkung von Ethyl-(2-amino-5,6-dichlor-2H-chinazolin-3-yl)-acetat-hydrobromid erwähnt.

Die Verbindung 6,7-Dichlor-1,5-dihydroimidazo-[2,1-b]-chinazolin-2-[3H]-on und deren Herstellung wurden erstmalig in US-A-3,932,407 beschrieben, worin eine Syntheseroute unter Verwendung von Phosphoroxychlorid via einer Anagrelidvorstufe, z.B. einem 2-Chloro-2H-chinazolin-Derivat, beschrieben wird, welche anschließend zur Anagrelid-Base, einer in Wasser schwerlöslichen Verbindung, zyklisiert wird. Zur Verbesserung der Wasserlöslichkeit werden solche Wirkstoffe in Salze umgewandelt, da sie oftmals nur in dieser Form vom Körper aufgenommen werden können. Der verwendete Syntheseweg zum Anagrelid wurde seitdem in einigen anderen Druckschriften bezüglich bestimmter Zwischenstufen modifiziert.

In "The Organic Chemistry of Drug Synthesis" (D. Lednicer und L.A. Mitscher, Wiley-Interscience; 1 edition (November 29, 1984) Vol. 3, S. 244, ISBN: 0471092509) wird eine Syntheseroute beschrieben, welche im Gegensatz zu US-A-3,932,407 mit einer 2,3-Dichlor-6-Nitrobenzylchlorid-Verbindung startet. Nach Alkylierung führt die Reduktion der Nitrogruppe zu einem Anilinderivat. Dieses wird zu einem Cyanamid umgesetzt, welches unter Freisetzung von Blausäure zum Chinazolin und weiter zum Anagrelid umgesetzt wird.

In US-A-4,146,718 und US-A-5,801,245 wird die vorherige Verwendung der gesundheitlich bedenklichen Verbindung Phosphoroxychlorid umgangen durch die Bereitstellung eines 2-Amino-2H-chinazolin-Derivates, welches anschließend in wasserfreiem Ethanol mit einer organischen Base unter mehrstündigem Rückflusskochen zur Anagrelid-Base umgesetzt wird.

US-A-5,391,737 beschreibt eine thermische Zyklisierung in einem salzsauren Medium zur Anagrelid-Base über ein Cyano-haltiges Chinazolin-Derivat.

In US-6,388,073 B1 ist ein alternativer Syntheseweg zur Herstellung von Anagrelid via 2,3-Dichlorbenzaldehyd als Ausgangsstoff offenbart. Diese alternative Syntheseroute führt ebenfalls zur bekannten Anagrelid-Vorstufe eines 2-Amino-2H-chinazolin-Derivates, welches anschließend bei Raumtemperatur im Wässrigen unter Verwendung einer organischen Base zyklisiert wird.

In WO 01/40196 werden 2-Amino-5,6-dihalogen-3,4-dihydrochinazoline offenbart, die zur Herstellung von Medikamenten eingesetzt werden, welche eine Blutplättchen reduzierende Wirkung aufweisen.

Allen Verfahren ist gemein, dass die Anagrelid-Base nach deren Erhalt gereinigt und anschließend in ein pharmazeutisch verträgliches Salz überführt werden muss. Organische Basen, wie Triethylamin oder Pyridin, müssen vollständig abgetrennt werden, da es sich hier um stark gesundheitsschädliche Verbindungen handelt. Ebenfalls ist die Überführung in das Salz ein sehr sensibler Schritt. Er führt systembedingt zu einer beginnenden sauren Hydrolyse des Lactam-Ringes des Anagrelids, die als nachteilig angesehen wird. Diese Hydrolyse findet auch nach Trocknung des Produktes weiterhin statt, was die Langzeitstabilität des Wirkstoffes negativ beeinflusst.

Die Inzidenz, an Polycythämia vera oder essentieller Thrombozythämie zu erkranken, liegt bei 0,5-1 auf 100.000 Individuen und Jahr. Polycythämia vera und essentielle Thrombozythämie gehören zu den relativ selten vorkommenden Krankheiten und werden daher auch als "Orphan diseases" bezeichnet. Auf Grund der begrenzten Anzahl potenzieller Patienten rechnet sich die Entwicklung spezifisch wirkender Arzneistoffe für Orphan diseases ökonomisch gar nicht. Sie sind gekennzeichnet durch hohe Entwicklungskosten bei gleichzeitig geringem Absatz. Die zudem hohen Produktionskosten von Anagrelid liegen oftmals in einer großen Anzahl von Synthesezwischenprodukten begründet, die mit mehr oder weniger geringen Ausbeuten hergestellt werden, wobei sich die Ausbeuten durch die Aufreinigungsschritte weiter verringern. Die Langzeitstabilität von Anagrelid wird zudem negativ beeinflusst durch die Notwendigkeit, diese Verbindung wegen der geringen Wasserlöslichkeit von Anagrelid-Base als pharmazeutisch verträgliches Hydrat-Hydrochlorid-Salz herzustellen, so dass es aufgrund der darin enthaltenen Chlorwasserstoffsäure und des Hydrat-Wassers zu einer langsam vorschreitenden Hydrolyse des Lactam-Ringes kommt.

Die der Erfindung zugrunde liegende Aufgabe besteht daher in der Bereitstellung eines neuen Wirkstoffes zur Behandlung myeloproliferativer Erkrankungen und zur Bronchodilation, wobei der Wirkstoff einfacher, preiswerter und in Bezug auf die Anforderungen an die Umweltverträglichkeit und die Produktionsbedingungen verträglich herstellbar sein soll. Ebenfalls soll das Problem der begrenzten Lagerzeitstabilität von Anagrelid aufgrund von Hydrolyse vermieden werden.

Die Aufgabe wird gelöst durch die Verwendung von 2-Amino-2H-chinazolin-Derivaten der allgemeinen Formel I und von deren pharmazeutisch verträglichen Salzen zur Herstellung von therapeutischen Mitteln zur Behandlung von myeloproliferativen Erkrankungen und zur Bronchodilation gemäß dem Anspruch 1.

Bevorzugte Ausführungsformen der Erfindung sind in den Unteransprüchen angegeben.

Die erfindungsgemäßen 2-Amino-2H-chinazolin-Derivate weisen folgende allgemeine chemische Formel I auf.

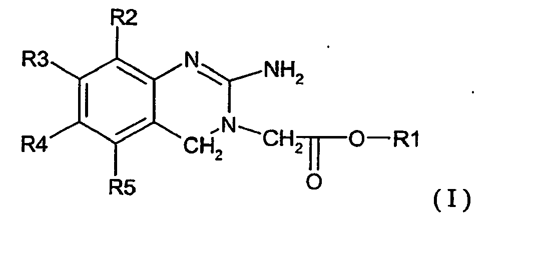

Darin sind R1 eine Alkylgruppe mit 1 - 5 Kohlenstoff-Atomen und R2, R3, R4 und R5 unabhängig voneinander jeweils ein Chlor- oder Wasserstoff-Atom. Die Alkylgruppen können geradkettige oder verzweigte Alkylgruppen mit 1 - 5 Kohlenstoff-Atomen sein, umfassend Methyl, Ethyl, n-Propyl, iso-Propyl, n-Butyl, sek-Butyl, tert-Butyl, n-Pentyl, sek-Pentyl, tert-Pentyl und neo-Pentyl. Bevorzugt sind Verbindungen, in denen R1 Methyl oder Ethyl ist. Weiter bevorzugt sind Verbindungen, in denen mindestens zwei Reste von R2, R3, R4 und R5 jeweils Chlor-Atome bedeuten. Besonders bevorzugt sind Verbindungen, in denen R1 für ein Methyl oder Ethyl steht, R2 und R3 jeweils ein Wasserstoff-Atom und R4 und R5 jeweils ein Chlor-Atom bedeuten.

Die vorliegende Erfindung umfasst auch pharmazeutisch verträgliche Salze der Verbindung mit der Formel I zur Anwendung insbesondere bei der Behandlung von myeloproliferativen Erkrankungen und zur Bronchodilation. Geeignete pharmazeutisch verträgliche Salze der Verbindung mit der Formel 1 sind Säureadditionssalze mit organischen und anorganischen Säuren, wie z.B. das Hydrochlorid, Hydrobromid, Sulfat, Fumarat, Maleat, Lactat und Succinat.

Bevorzugte 2-Amino-2H-chinazolin-Derivate zur Lösung der Aufgabe sind Methyl-(2-amino-5,6-dichlor-3,4-dihydro-2H-chinazolin-3-yl)-acetat-hydrochlorid-hemihydrat und Ethyl-(2-amino-5,6-dichlor-3,4-dihydro-2H-chinazolin-3-yl)-acetat-hydrochlorid-hemihydrat sowie Methyl-(2-amino-5,6-dichlor-3,4-dihydro-2H-chinazolin-3-yl)-acetat-hydrobromid-hemihydrat und Ethyl-(2-amino-5,6-dichlor-3,4-dihydro-2H-chinazolin-3-yl)-acetat-hydrobromid-hemihydrat. Das enthaltene Kristallwasser kann auch in anderen Molverhältnissen vorliegen oder gegebenenfalls auch nicht vorhanden sein.

Die Synthese der Ausgangsverbindungen der allgemeinen Formel I kann beispielsweise durch die Syntheseroute aus obigen Druckschriften US-A-4,146,718 oder US-A-6,388,073 durchgeführt werden.

Bevorzugt wird eine Syntheseroute verwendet, welche der Syntheseroute in dem oben genannten Handbuch "The Organic Chemistry of Drug Synthesis" (D. Lednicer und L.A. Mitscher, Wiley-Interscience; 1 edition (November 29, 1984) Vol. 3, S. 244) entnommen ist.

Die Herstellung der Salze kann in bekannter Art und Weise durch Zugabe der entsprechenden Säure zu einer methanolischen Aufschlämmung eines 2-Amino-2H-chinazolin-Derivates der Formel I erfolgen. Ebenfalls ist eine Salzbildung im Magen von Säugetieren denkbar, analog der Salzbildung von Anagrelid in US-A-6,388,073.

Die erfindungsgemäßen 2-Amino-2H-chinazolin-Derivate können im wässrigen Medium in Abhängigkeit vom pH-Wert ohne Zugabe von weiteren Hilfsstoffen oder stundenlanges Kochen unter Rückfluss zum Anagrelid zyklisieren. Durch die Zyklisierung in wässriger schwach alkalischer Lösung können bereits die erfindungsgemäßen Derivate der allgemeinen Formel I, worin R1 eine Alkylgruppe mit 1 - 5 Kohlenstoff-Atomen und R2, R3, R4 und R5 unabhängig voneinander jeweils ein Chlor- oder Wasserstoff-Atom bedeuten, direkt ohne weiteren Syntheseaufwand als so genannte Prodrug-Verbindungen zur Anwendung insbesondere bei myeloproliferativen Erkrankungen bei Säugetieren und vor allem beim Menschen eingesetzt werden, da sich herausgestellt hat, dass der vorherrschende pH-Wert im Säugetier, insbesondere im Darm, von 8 - 10 zu einer *in vivo -* Zyklisierung dieser Verbindung und damit zur Bildung des Wirkstoffes Anagrelid führt. Eine Zyklisierung der Verbindung mit der allgemeinen chemischen Formel I im stark sauren pH-Bereich, wie er beispielsweise im Magen von Säugetieren vorherrscht, wurde nur zu einem geringen Prozentsatz nachgewiesen.

Wenn der Begriff "Prodrug" verwendet wird, bezieht er sich auf eine Verbindung der allgemeinen Formel I oder auf deren pharmazeutisch verträgliche Salze, da diese eine unmittelbare Vorstufe zum Wirkstoff Anagrelid darstellen und unter den gegebenen Umständen durch Zyklisierung zu der eigentlichen, aus dem Stand der Technik bekannten Wirkstoffsubstanz Anagrelid oder eines Anagrelid-Derivates führen.

Der beschriebene Einsatz von gesundheitsschädlichen organischen Basen, wie z.B. Triethylamin oder Pyridin, zur Zyklisierung der erfindungsgemäßen Verbindung bei der Herstellung von Anagrelid entfällt. Dieses Ergebnis führt zu einer Einsparung der weiteren Syntheseschritte zur Herstellung von Anagrelid und Anagrelid-Salz und deren nachgeschaltete Aufreinigung und damit zu einer höheren Produktionsausbeute. Zusätzlich wird das Problem der Hydrolyse des Wirkstoffes in Salzform ausgeschlossen.

Das therapeutische Mittel ist ein pharmazeutisches Präparat, das als Wirkstoff wenigstens eine Verbindung der allgemeinen Formel I, gegebenenfalls weitere Wirkstoffe sowie mindestens einen pharmazeutischen Hilfsstoff enthalten. Vorzugsweise werden diese Mittel zur Behandlung von myeloproliferativen Erkrankungen, wie essentieller Thrombozythämie und Polycythämia vera (in Verbindung mit Thrombozytose), eingesetzt. Weiterhin kann das erfindungsgemäße Präparat zur Bronchodilation eingesetzt werden. Entsprechende pharmazeutische Formulierungen werden aus mindestens einer Verbindung der allgemeinen Formel I und/oder deren pharmazeutisch verträglichen Salzen und pharmazeutisch verträglichen Hilfsstoffen, insbesondere Trägermaterialien, Lösungsmitteln, Füllstoffen, Verdünnungsmitteln, Farbstoffen und/oder Bindemitteln, in an sich bekannter Weise zubereitet bzw. hergestellt. Die Auswahl der eingesetzten festen oder flüssigen Hilfsstoffe sowie deren Mengen hängen davon ab, wie das Arzneimittel verabreicht wird.

Bevorzugte Zubereitungen bestehen in einer Darreichungsform, die zur oralen, enteralen, intravenösen (i.v.) oder örtlichen Applikation geeignet ist. Solche Darreichungsformen sind beispielsweise Tabletten, Filmtabletten, Dragees, Pillen, Kapseln, Pulver, Flüssigkeiten, wie Sirupe, Gele, injizierbare Flüssigkeiten, zur intravenösen Injektion usw. Weiterhin sind auch Depotformen, wie implantierbare Zubereitungen, sowie Suppositorien geeignet. Dabei geben die einzelnen Zubereitungen die erfindungsgemäßen Derivate je nach deren Art allmählich oder die gesamte Menge in kurzer Zeit an den Körper ab.

Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben dem mindestens einen 2-Amino-2H-chinazolin-Derivat und/oder dessen pharmazeutisch verträglichen Salzen einen oder mehrere pharmazeutisch verträgliche Hilfsstoffe enthalten, beispielsweise Stoffe zur Einstellung der Rheologie des Arzneimittels, oberflächenaktive Stoffe, Lösungsvermittler, Mikrokapseln, Mikropartikel, Granulate, Verdünner, Bindemittel, wie Stärke, Zucker, Sorbit und Gelatine, ferner Füllstoffe, wie Kieselsäure und Talkum, Gleitmittel, Farbstoffe, Duftstoffe und andere Stoffe.

Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog zu den Tabletten hergestellten Kemen mit üblicherweise in Dragéeüberzügen verwendeten Mitteln, beispielsweise Polyvinylpyrrolidon oder Schellack, Gummiarabicum, Talk, Titanoxid oder Zucker, hergestellt werden. Dabei kann auch die Dragéehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Die erfindungsgemäßen 2-Amino-2H-chinazolin-Derivate können auch in Form einer Lösung formuliert werden, die für die orale Verabreichung bestimmt ist und die neben dem aktiven Derivat als Bestandteile mindestens ein pharmazeutisch verträgliches Öl und/oder mindestens eine pharmazeutisch verträgliche lipophile, oberflächenaktive Substanz und/oder mindestens eine pharmazeutisch verträgliche hydrophile, oberflächenaktive Substanz und/oder mindestens ein pharmazeutisch verträgliches wassermischbares Lösungsmittel enthält.

Die erfindungsgemäßen Substanzen können auch in geeigneten Lösungen, wie beispielsweise physiologischer Kochsalzlösung, als Infusions- oder Injektionslösung zur Anwendung kommen. Für die parenterale Applikation können die Wirkstoffe in mindestens einem physiologisch verträglichen Verdünnungsmittel gelöst oder suspendiert sein. Zur Erhöhung der Löslichkeit können Lösungsvermittler zugesetzt werden.

Zur Formulierung eines injizierbaren Präparats kann ein beliebiger flüssiger Träger verwendet werden, in dem die erfindungsgemäßen Verbindungen gelöst oder emulgiert sind. Diese Flüssigkeiten enthalten häufig auch Stoffe zur Regulation der Viskosität, oberflächenaktive Stoffe, Konservierungsstoffe, Lösungsvermittler, Verdünner und weitere Zusatzstoffe, mit denen die Lösung isotonisch eingestellt wird. Zusammen mit den erfindungsgemäßen Derivaten können auch andere Wirkstoffe verabreicht werden.

Ebenfalls können die erfindungsgemäßen Substanzen Hilfsstoffe enthalten, welche in wässrigen Lösungen basisch reagieren, so dass die Zyklisierung der erfindungsgemäßen Substanzen erfolgen kann, beispielsweise in einem Inhalationsbad oder in einer Spray-Lösung zur Bronchodilation oder in einem Depot innerhalb oder außerhalb des Körpers des Säugetieres.

Die Dosierung der erfindungsgemäßen Derivate der allgemeinen Formel I wird vom behandelnden Arzt bestimmt und hängt unter anderem vom Gewicht des Patienten, der Indikation, der Applikationsart und dem Schweregrad der Erkrankung ab. Die tägliche Dosis beträgt nicht mehr als 5 mg, üblicherweise 0,5 bis 5,0 mg, wobei die Dosis als einmal zu verabreichende Einzeldosis oder unterteilt in zwei oder mehrere Tagesdosen gegeben werden kann.

Die folgenden Beispiele dienen der Erläuterung der Erfindung, schränken aber den allgemeinen Erfindungsgedanken nicht ein. Die Ausbeuten der hergestellten Verbindungen sind nicht optimiert. Die Temperaturen sind nicht korrigiert.

Die in den folgenden Beispielen verwendeten Ethyl- und Methyl-(2-amino-5,6-dichlor-2H-chinazolin-3-yl)-acetat-Derivate wurden jeweils beginnend mit 2,3-Dichlor-6-nitrobenzylchlorid mittels folgender dreistufiger Synthese hergestellt:

### A) Ethyl-(2-amino-5,6-dichlor-2H-chinazolin-3-yl)-acetat-hydrobromid (MW 383,1)

### 1. Stufe - N-(2,3-Dichlor-6-nitrobenzyl)-glycinethylester-hydrochlorid (MW 343,6)

In einem 10l-Reaktionskolben wurden bei Raumtemperatur als Reaktionsmischung 818,5 g 2,3-Dichlor-6-nitrobenzylchlorid in 3700 ml Acetonitril unter Rühren vorgelegt und 724,5 g Glycerinethylester-hydrochlorid zugeben. Zu dieser Reaktionsmischung wurden unter Rühren innerhalb von 30-50 min 2415 ml Triethylamin zugetropft. Dann wurde die Reaktionsmischung bei zirka 75°C unter Rückfluss für 2 h gekocht und die Umsetzung mittels HPLC verfolgt. Anschließend wurde auf Raumtemperatur abgekühlt.

Zur Gewinnung des Reaktionsproduktes wurde die flüssige Phase vom ausgefallenen Triethylamin*HCl abgetrennt, wobei das ausgefallene Triethylamin*HCl mit zirka 1800 ml Acetonitril gewaschen wurde. Waschphase und flüssige Phase wurden vereint und am Rotationsverdampfer zur Trockne eingeengt. Der Rückstand wurde bei zirka 5°C mit Tetrahydrofuran (THF) gewaschen. Die flüssige Phase wurde vom ausgefallenen Triethylamin*HCl abgetrennt, das ausgefallene Triethylamin*HCl mit THF nachgewaschen und die vereinten organischen Phasen am Rotationsverdampfer zur Trockne eingeengt. Das so erhaltene Reaktionsprodukt wurde in der dreifachen Gewichtsmenge Ethanol unter Rühren bei Raumtemperatur gelöst und mit 5-6 N Salzsäure (in 2-Propanol) versetzt. Der Ansatz wurde auf zirka 5°C abgekühlt, das ausgefallene Rohprodukt abgesaugt, mit Ethanol gewaschen und im Trockenschrank bei zirka 60°C getrocknet. Die Ausbeute des Reaktionsproduktes N-(2,3-Dichlor-6-nitrobenzyl)-glycinethylester-hydrochlorid betrug 911 g (72 % d.Th. (Gew.-%)).

### 2. Stufe - N-(6-Amino-2,3-dichlorbenzyl)-glycinethylester-dihydrochlorid (MW 350,1)

In einem 20l-Reaktionskolben wurden bei Raumtemperatur 1048 g N-(2,3-Dichlor-6-nitrobenzyl)-glycinethylester-hydrochlorid in 11980 ml Ethanol unter Rühren suspendiert und 700 ml 5-6 N Salzsäure (in 2-Propanol) zugeben. Anschließend wurde die gesamte Reaktionsapparatur mit Stickstoff gespült. Zu dieser Reaktionsmischung wurden 104 g Palladium auf Aktivkohle (10 % Palladium) zugesetzt, welches zuvor in zirka 300 ml Ethanol aufgeschlämmt wurde. Nach dem Evakuieren der Reaktionsapparatur wurde Wasserstoff zugeführt und die Hydrierung bei 0,3 bar durchgeführt, bis zirka 97 % des Ausgangsstoffes umgesetzt waren. Während der Hydrierung erwärmte sich die Reaktionsmischung. Die Umsetzung wurde mittels HPLC verfolgt. Anschließend wurde die noch warme Reaktionsmischung über einen auf zirka 80°C vorgewärmten Filter abgesaugt. Der Katalysator wurde mit zirka 600 ml Ethanol gewaschen. Die flüssigen Phasen wurden vereint, auf 4 - 8°C unter Rühren abgekühlt. Das ausgefallene Endprodukt Dihydrochlorid wurde abgesaugt, mit 300 ml Ethanol gewaschen und im Trockenschrank bei zirka 60°C getrocknet. Die Ausbeute des Produktes N-(2,3-Dichlor-6-nitrobenzyl)-glycinethylester-hydrochlorid betrug 726 g (68 % d.Th. (Gew.-%)).

### 3. Stufe - Ethyl-(2-amino-5,6-dichlor-2H-chinazolin-3-yl)-acetat-hydrobromid (Endprodukt)

In einem 10l-Reaktionskolben wurden bei Raumtemperatur 940 g N-(6-Amino-2,3-Dichlorbenzyl)-glycinethylester-dihydrochlorid in 4950 ml Ethanol unter Rühren suspendiert und auf zirka 8°C abgekühlt. Anschließend wurden zirka 1733 ml Natriumethylat-Lösung (20 Gew-% NaOEt) langsam zugeführt und ein pH-Wert zwischen 11,2 und 11,8 eingestellt. Das entstehende Natriumchlorid wurde so lange abgesaugt bis die Reaktionsmischung klar wurde. Zu der klaren Reaktionsmischung wurden unter Rühren 2227 ml Bromcyan-Lösung (c=250 g/L in Ethanol) zugegeben. Die Reaktionsmischung wurde nach Zugabe zirka 18 h bei Raumtemperatur gerührt und der Umsatz zum Endprodukt mittels HPLC kontrolliert. Die Ausbeute des Endproduktes N-(2,3-Dichlor-6-nitrobenzyl)-glycinethylester-hydrochlorid betrug 770 g (75 % d.Th. (Gew.-%)).

Zur Gewinnung des Endproduktes wurde die Reaktionsmischung auf zirka 5°C abgekühlt, das ausgefallene Endprodukt abgesaugt und mit Ethanol gewaschen. Anschließend wurde das Endprodukt im Trockenschrank getrocknet.

### B) Methyl-(2-amino-5,6-dichlor-2H-chinazofin-3-yl)-acetat-hydrochlorid (MW 324,6)

### 1. Stufe - N-(2,3-Dichlor-6-nitrobenzyl)-glycinmethylester-hydrochlorid (MW 329,6)

Die Reaktion wurde, wie unter A) 1. Stufe beschrieben, durchgeführt, wobei anstelle von Glycerinethylester-hydrochlorid Glycerinmethylester-hydrochlorid eingesetzt wurde. Die Ausbeute des Rohproduktes betrug 903 g (73 % d.Th. (Gew.%)).

2. Stufe - N-(6-Amino-2,3-dichlorbenzyl)-glycinmethylester-dihydrochlorid (MW 336,1) Die Reaktion wurde, wie unter A) 2. Stufe beschrieben, durchgeführt, wobei anstelle von Glycerinethylester-hydrochlorid Glycerinmethylester-hydrochlorid eingesetzt wurde. Die Ausbeute des Produktes betrug 740 g (62 % d.Th. (Gew.-%)).

### 3. Stufe - Methyl-(2-amino-5,6-dichlor-2H-chinazolin-3-yl)-acetat-hydrochlorid (Endprodukt)

Die Reaktion wurde, wie unter A) 3. Stufe beschrieben, durchgeführt, wobei anstelle von Glycerinethylester-dihydrochlorid Glycerinmethylester-dihydrochlorid eingesetzt wurde. Zur Umsetzung wurde eine Chlorcyan-Lösung (c=145 g/L in Ethanol) verwendet, wobei die Reaktion in einem geschlossenen Reaktionsbehälter bei Raumtemperatur durchgeführt wurde.
Die Ausbeute des Rohproduktes betrug 650 g (75 % d.Th. (Gew.-%)).

### Vergleichsbeispiel 1

### Methyl-(2-amino-5,6-dichlor-2H-chinazolin-3-yl)-acetat-hydrochlorid

Zu 10 ml einer wässrigen salzsauren Lösung (0,1 M HCl) wurden zur Zyklisierung 0,3 mmol des Anagrelid - Prodrugs Methyl-(2-amino-5,6-dichlor-2H-chinazolin-3-yl)-acetat-hydrochlorid (Analyse UV-Maxima 217, 262 nm; MW 324,59; Smp. >250 °C), entsprechend der allgemeinen Formel I, worin R1 eine Alkylgruppe mit einem Kohlenstoff-Atom, R2 und R3 jeweils ein Wasserstoff-Atom und R4 und R5 jeweils ein Chlor-Atom bedeuten, bei 37°C unter leichtem Rühren zugegeben. Nach einer Stunde waren 9,6 % des Prodrugs zu Anagrelid umgesetzt.

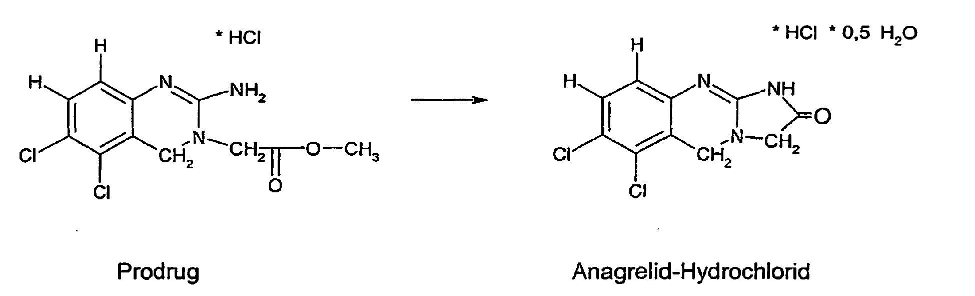

### Vergleichsbeispiel 2

### Methyl-(2-amino-5,6-dichlor-2H-chinazolin-3-yl)-acetat-hydrochlorid

Zu 10 ml pH-neutralem Wasser wurden zur Zyklisierung 0,3 mmol des Anagrelid - Prodrugs Methyl-(2-amino-5,6-dichlor-2H-chinazolin-3-yl)-acetat-hydrochforid (MW 324,59), entsprechend der allgemeinen Formel I, worin R1 eine Alkylgruppe mit einem Kohlenstoff-Atom, R2 und R3 jeweils ein Wasserstoff-Atom und R4 und R5 jeweils ein Chlor-Atom bedeuten, bei 37°C unter leichtem Rühren zugegeben. Nach einer Stunde waren 58 % des Prodrugs zu Anagrelid umgesetzt.

### Beispiel 1

### Methyl-(2-amino-5,6-dichlor-2H-chinazolin-3-yl)-acetat-hydrochlorid

Zu 10 ml einer wässrigen basischen Lösung (0,1 M NaOH) wurden zur Zyklisierung 0,3 mmol des Anagrelid - Prodrugs Methyl-(2-amino-5,6-dichlor-2H-chinazolin-3-yl)-acetat-hydrochlorid, entsprechend der allgemeinen Formel I, worin R1 eine Alkylgruppe mit einem Kohlenstoff-Atom, R2 und R3 jeweils ein Wasserstoff-Atom und R4 und R5 jeweils ein Chlor-Atom bedeuten, bei 37°C unter leichtem Rühren zugegeben. Nach einer Stunde waren 100 % des Prodrugs zu Anagrelid umgesetzt.

### Beispiel 2

### Ethyl-(2-amino-5,6-dichlor-2H-chinazolin-3-yl)-acetat-hydrochlorid-hemihydrat

Zu 10 ml einer wässrigen basischen Lösung (15g Natriumhydrogencarbonat in 1l Wasser) wurden zur Zyklisierung 0,3 mmol des Anagrelid - Prodrugs Ethyl-(2-amino-5,6-dichlor 2H-chinazolin-3-yl)-acetat-hydrochlorid-hemihydrat, entsprechend der allgemeinen Formel I, worin R1 eine Alkylgruppe mit zwei Kohlenstoff-Atomen, R2 und R3 jeweils ein Wasserstoff-Atom und R4 und R5 jeweils ein Chlor-Atom bedeuten, bei 37°C unter leichtem Rühren zugegeben. Diese basische Lösung wurde so gewählt, dass sie einer üblichen Versuchsanordnung zur Simulation des Darmsaft-Milieus (pH-Wert zwischen 8 und 10) entspricht. Nach einer Stunde waren 100 % des Prodrugs zu Anagrelid umgesetzt.

### Beispiel 3

### Methyl-(2-amino-5,6-dichlor-2H-chinazolin-3-yl)-acetat-hydrochlorid-hemihydrat

Zu 10 ml einer wässrigen basischen Lösung (15g Natriumhydrogencarbonat in 1l Wasser) wurden zur Zyklisierung 0,3 mmol des Anagrelid - Prodrugs Methyl-(2-amino-5,6-dichlor-2H-chinazolin-3-yl)-acetat-hydrochlorid-hemihydrat, entsprechend der allgemeinen Formel I, worin R1 eine Alkylgruppe mit einem Kohlenstoff-Atom, R2 und R3 jeweils ein Wasserstoff-Atom und R4 und R5 jeweils ein Chlor-Atom bedeuten, bei 37°C unter leichtem Rühren zugegeben. Diese basische Lösung wurde so gewählt, dass sie einer üblichen Versuchsanordnung zur Simulation des Darmsaft-Milieus (pH-Wert zwischen 8 und 10) entspricht. Nach einer Stunde waren 100 % des Prodrugs zu Anagrelid umgesetzt.

### Beispiel 4

### Ethyl-(2-amino-5,6-dichlor-2H-chinazolin-3-yl)-acetat-hydrobromid

Zu 10 ml einer wässrigen basischen Lösung (15g Natriumhydrogencarbonat in 1l Wasser) wurden zur Zyklisierung 0,3 mmol des Anagrelid - Prodrugs Ethyl-(2-amino-5,6-dichlor-2H-chinazolin-3-yl)-acetat-hydrobromid (Analyse UV-Maxima 204, 216, 262 nm; Smp. 291-293), entsprechend der allgemeinen Formel I, worin R1 eine Alkylgruppe mit zwei Kohlenstoff-Atomen, R2 und R3 jeweils ein Wasserstoff-Atom und R4 und R5 jeweils ein Chlor-Atom bedeuten, bei 37°C unter leichtem Rühren zugegeben. Diese basische Lösung wurde so gewählt, dass sie einer üblichen Versuchsanordnung zur Simulation des Darmsaft-Milieus (pH-Wert zwischen 8 und 10) entspricht. Nach einer Stunde waren 100 % des Prodrugs zu Anagrelid umgesetzt.

### Beispiel 5 - In vivo Versuch

### Methyl-(2-amino-5,6-dichlor-3,4-dihydro-2H-chinazolin-3-yl)-acetat-hydrochlorid

Im folgenden Beispiel wurde im Gegensatz zu den Beispielen 1-4 das Anagrelid-Prodrug Methyl-(2-amino-5,6-dichlor-3,4-dihydro-2H-chinazolin-3-yl)-acetat-hydrochlorid in verschieden Konzentrationen im Tierversuch (in vivo) an 6 Ratten hinsichtlich der Absorption und des Blutspiegelverlaufs des gebildeten Anagrelids nach einmaliger oraler Gabe untersucht.

Dazu wurde das Prodrug in destilliertem Wasser gelöst und den Versuchstieren (jeweils im Doppelversuch) mittels Magensonde oral verabreicht. Die Pharmakokinetik wurde durch Blutentnahme aus dem retroorbitalen Venenplexus 1, 2, 4 und 8 Stunden nach der Substanzverabreichung ermittelt. Dazu wurden die Konzentrationen von Anagrelid und seiner Abbauprodukte in den Blutproben mittels HPLC-MS bestimmt. Die Ergebnisse sind in folgender Tab. 1 gezeigt.

**Tab. 1 Konzentration des Anagrelids und seiner Abbauprodukte im Blut von Ratten, gebildet aus dem Anagrelid-Prodrug Methyl-(2-amino-5,6-dichlor-3,4-dihydro-2H-chinazolin-3-yl)-acetat-hydrochlorid**

| Lfd. Nr. | Versuchstier | Dosis Prodrug [mg/kg] | Zeit [h] | Anagrelid [ng/ml] | Anagrelid-Abbauprodukte [ng/ml] |
|---|---|---|---|---|---|
| 1 | 11 | 1 | 1 | 88,7 | u.N. |
| 2 | | | 2 | u.N. | u.N. |
| 3 | | | 4 | u.N. | u.N. |
| 4 | | | 8 | u.N. | u.N. |
| 5 | 12 | 1 | 1 | 81,7 | u.N. |
| 6 | | | 2 | u.N. | u.N. |
| 7 | | | 4 | u.N. | u.N. |
| 8 | | | 8 | u.N. | u.N. |
| 9 | 21 | 3 | 1 | 684,7 | u.N. |
| 10 | | | 2 | 513,6 | u.N. |
| 11 | | | 4 | 127,2 | u.N. |
| 12 | | | 8 | u.N. | u.N. |
| 13 | 22 | 3 | 1 | 710,3 | u.N. |
| 14 | | | 2 | 483,8 | u.N. |
| 15 | | | 4 | 153,5 | u.N. |
| 16 | | | 8 | u.N. | u.N. |
| 17 | 31 | 10 | 1 | 4176,2 | 44,9 |
| 18 | | | 2 | 4051,0 | u.N. |
| 19 | | | 4 | 1828,2 | u.N. |
| 20 | | | 8 | 514,4 | u.N. |
| 21 | 32 | 10 | 1 | 6079,1 | 41,2 |
| 22 | | | 2 | 5859,2 | u.N. |
| 23 | | | 4 | 4023,6 | 53,5 |
| 24 | | | 8 | 1918,3 | 61,3 |

| | | | | | |
|---|---|---|---|---|---|
| u.N. - Wert lag unterhalb der Nachweisgrenze von 40 ng/ml. | | | | | |

Die Ergebnisse zeigen, dass Anagrelid in Abhängigkeit von der eingesetzten Konzentration des Prodrugs nach Verabreichung schnell im Körper der Tiere gebildet wird und anschließend im Blut der Tiere nachweisbar ist. Die Konzentrationsverläufe des gebildeten Anagrelids und die Bildung typischer Abbauprodukte zeigen, dass das gebildete Anagrelid auch metabolisiert wird. Bei höheren Ausgangskonzentrationen des Prodrugs können auch typische Abbauprodukte des Anagrelids nachgewiesen werden. Das Auftreten von Anagrelid und Anagrelid-Abbauprodukten im Blut zeigt, dass Anagrelid-Prodrug geeignet ist, als Vorstufe des Anagrelids eingesetzt zu werden. Die *in vivo* Versuche stützen dabei die Ergebnisse der Beispiele 1-4.

## Patentansprüche

1. Verwendung von 2-Amino-2H-chinazolin-Derivaten der allgemeinen chemischen Formel I 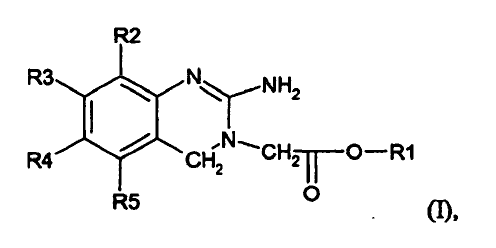 worin R1 eine Alkylgruppe mit 1 - 5 Kohlenstoff-Atomen und R2. R3, R4 und R5 unabhängig voneinander jeweils ein Chlor- oder Wasserstoff-Atom bedeuten, sowie von deren pharmazeutisch verträglichen Salzen, zur Herstellung von therapeutischen Mitteln zur Behandlung von myeloproliferativen Erkrankungen und zur Bronchodilation bei Säugetieren.

2. Verwendung nach Anspruch 1, **dadurch gekennzeichnet, dass** mindestens zwei Reste von R2, R3, R4 und R5 jeweils Chlor-Atome bedeuten.

3. Verwendung nach einem der vorstehenden Ansprüche, **dadurch gekennzeichnet, dass** R1 für Methyl oder Ethyl steht, R2 und R3 jeweils ein Wasserstoff-Atom und R4 und R5 jeweils ein Chlor-Atom bedeuten.

4. Verwendung nach einem der vorstehenden Ansprüche, **dadurch gekennzeichnet, dass** die pharmazeutisch verträglichen Salze ausgewählt sind aus der Gruppe, umfassend das Hydrochlorid, Hydrobromid, Sulfat, Fumarat, Maleat, Lactat und Succinat.

5. Verwendung nach einem der vorstehenden Ansprüche, **dadurch gekennzeichnet, dass** die 2-Amino-2H-chinazolin-Derivate ausgewählt sind aus der Gruppe, umfassend Methyl-(2-amino-5,6-dichlor-3,4-dihydro-2H-chinazolin-3-yl)-acetat-hydrochlorid-hemihydrat, Methyl-(2-amino-5,6-dichlor-3,4-dihydro-2H-chinazolin-3-yl)-acetat-hydrobromid-hemihydrat, Ethyl-(2-amino-5,6-dichlor 3,4-dihydro-2H-chinazolin-3-yl)-acetat-hydrochlorid-hemihydrat, und Ethyl-(2-amino-5,6-dichlor-3,4-dihydro-2H-chinazolin-3-yl)-acetat-hydrobromid-hemihydrat.

6. Verwendung eines pharmazeutischen Präparates, enthaltend mindestens ein Mittel, ausgewählt aus der Gruppe, umfassend 2-Amino-2H-chinazolin-Derivate der allgemeinen chemischen Formel 1 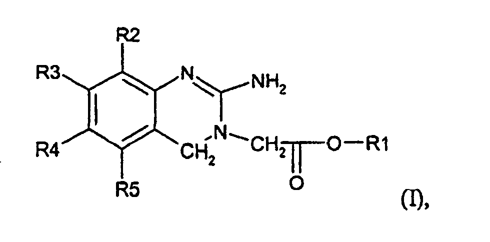 worin R1 eine Alkylgruppe mit 1 - 5 Kohlenstoff-Atomen und R2, R3, R4 und R5 unabhängig voneinander jeweils ein Chlor- oder Wasserstoff-Atom bedeuten und von deren pharmazeutisch verträglichen Salzen, und mindestens einen pharmazeutisch verträglichen Hilfsstoff, zur Herstellung von therapeutischen Mitteln zur Behandlung von myeloproliferativen Erkrankungen und zur Bronchodilation bei Säugetieren.

7. Verwendung des pharmazeutisches Präparates nach Anspruch 6, **dadurch gekennzeichnet, dass** mindestens einer der pharmazeutisch verträglichen Hilfsstoffe in wässrigem Medium basisch reagiert.

## Claims

1. Use of 2-amino-2H-quinazoline derivatives of the general chemical formula I 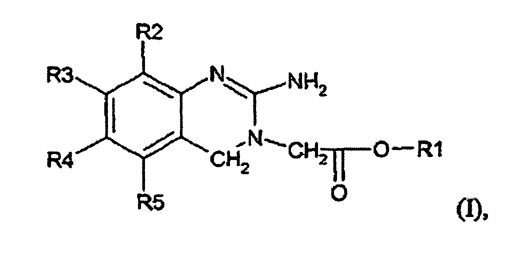 wherein R1 is an alkyl group with 1-5 carbon atoms and R2, R3, R4 and R5, independently of one another, each indicate a chlorine or hydrogen atom, as well as their pharmaceutically compatible salts, for producing therapeutic agents for the treatment of myeloproliferative diseases and for bronchodilation in mammals.

2. The use according to claim 1, further **characterized in that** at least two residues of R2, R3, R4 and R5 each indicate chlorine atoms.

3. The use according to one of the preceding claims, further **characterized in that** R1 stands for methyl or ethyl, R2 and R3 are each hydrogens, and R4 and R5 are each chlorine atoms.

4. The use according to one of the preceding claims, further **characterized in that** the pharmaceutically compatible salts are selected from the group comprising the hydrochloride, hydrobromide, sulfate, fumarate, maleate, lactate and succinate.

5. The use according to one of the preceding claims, further **characterized in that** the 2-amino-2H-quinazoline derivatives are selected from the group comprising methyl-(2-amino-5,6-dichloro-3,4-dihydro-2H-quinazolin-3-yl) acetate hydrochloride hemihydrate, methyl-(2-amino-5,6-dichloro-3,4-dihydro-2H-quinazolin-3-yl) acetate hydrobromide hemihydrate, ethyl-(2-amino-5,6-dichloro-3,4-dihydro-2H-quinazolin-3-yl) acetate hydrochloride hemihydrate, und ethyl-(2-amino-5,6-dichloro-3,4-dihydro-2H-quinazolin-3-yl) acetate hydrobromide hemihydrate.

6. Use of a pharmaceutical preparation containing at least one agent, selected from the group comprising 2-amino-2H-quinazoline derivatives of the general chemical formula I 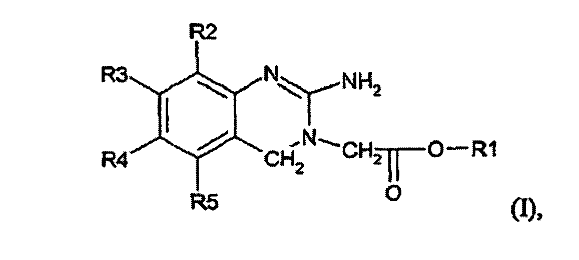 wherein R1 is an alkyl group with 1-5 carbon atoms and R2, R3, R4 and R5, independently of one another, each indicate a chlorine or hydrogen atom, and their pharmaceutically compatible salts, and at least one pharmaceutically compatible adjuvant, for producing therapeutic agents, for the treatment of myeloproliferative diseases and for bronchodilation in mammals.

7. The use of the pharmaceutical preparation according to claim 6, further **characterized in that** at least one of the pharmaceutically compatible adjuvants reacts basically in aqueous medium.

## Revendications

1. Utilisation de dérivés de 2-amino-2H-quinazoline de formule chimique générale I 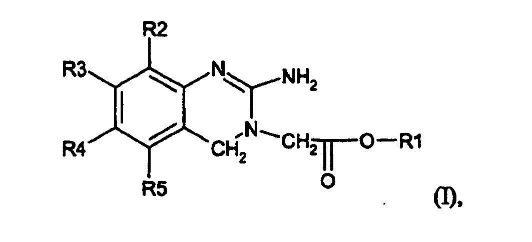 dans laquelle R1 signifie un groupe alkyle comprenant 1 à 5 atomes de carbone et R2, R3, R4 et R5 signifient, indépendamment l'un de l'autre, à chaque fois un atome de chlore ou d'hydrogène, ainsi que de leurs sels pharmaceutiquement acceptables pour la préparation d'agents thérapeutiques destinés au traitement de maladies myéloprolifératives et pour la bronchodilatation chez les mammifères.

2. Utilisation selon la revendication 1, **caractérisée en ce qu'**au moins deux radicaux parmi R2, R3, R4 et R5 signifient à chaque fois des atomes de chlore.

3. Utilisation selon l'une quelconque des revendications précédentes, **caractérisée en ce que** R1 représente méthyle ou éthyle, R2 et R3 signifient à chaque fois un atome d'hydrogène et R4 et R5 signifient à chaque fois un atome de chlore.

4. Utilisation selon l'une quelconque des revendications précédentes, **caractérisée en ce que** les sels pharmaceutiquement acceptables sont choisis dans le groupe comprenant le chlorhydrate, le bromhydrate, le sulfate, le fumarate, le maléate, le lactate et le succinate.

5. Utilisation selon l'une quelconque des revendications précédentes, **caractérisée en ce que** les dérivés de 2-amino-2H-quinazoline sont choisis dans le groupe comprenant le chlorhydrate hémi-hydraté de (2-amino-5,6-dichloro-3,4-dihydro-2H-quinazolin-3-yl)-acétate de méthyle, le bromhydrate hémi-hydraté de (2-amino-5,6-dichloro-3,4-dihydro-2H-quinazolin-3-yl)-acétate de méthyle, le chlorhydrate hémi-hydraté de (2-amino-5,6-dichloro-3,4-dihydro-2H-quinazolin-3-yl)-acétate d'éthyle, et le bromhydrate hémi-hydraté de (2-amino-5,6-dichloro-3,4-dihydro-2H-quinazolin-3-yl)-acétate d'éthyle.

6. Utilisation d'une préparation pharmaceutique, contenant au moins un agent choisi dans le groupe comprenant les dérivés de 2-amino-2H-quinazoline de formule chimique générale I 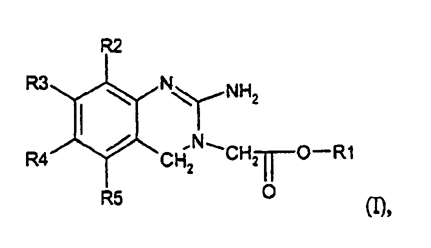 dans laquelle R1 signifie un groupe alkyle comprenant 1 à 5 atomes de carbone et R2, R3, R4 et R5 signifient, indépendamment l'un de l'autre, à chaque fois un atome de chlore ou d'hydrogène, ainsi que leurs sels pharmaceutiquement acceptables, et au moins un adjuvant pharmaceutiquement acceptable pour la préparation d'agents thérapeutiques destinés au traitement de maladies myéloprolifératives et pour la bronchodilatation chez les mammifères.

7. Utilisation d'une préparation pharmaceutique selon la revendication 6, **caractérisée en ce qu'**au moins un des adjuvants pharmaceutiquement acceptables réagit par voie basique en milieu aqueux.
